# EUROPEAN PATENT SPECIFICATION

(11) **EP 2 747 817 B1**
(45) Date of publication and mention of the grant of the patent: **10.10.2018**
(21) Application number: 12778417.1
(22) Date of filing: 14.08.2012
(51) Int. Cl.: A61M 16/00

(54) **APPARATUS FOR CONTROLLING A VENTILATION THERAPY DEVICE**
VORRICHTUNG ZUR STEUERUNG EINER ATEMTHERAPIEVORRICHTUNG
APPAREIL DE GESTION D'UN DISPOSITIF THÉRAPEUTIQUE DE VENTILATION

(30) Priority: 25.08.2011 US 201161527330 P
(43) Date of publication of application: 02.07.2014
(73) Proprietor: Koninklijke Philips N.V., 5656 AE Eindhoven (NL)
(72) Inventor: GARDE, Smita, 5656 AE Eindhoven (NL); AHMAD, Samir, 5656 AE Eindhoven (NL)
(74) Representative: Steffen, Thomas
(86) International application number: PCT/IB2012/054119
(87) International publication number: WO 2013/027151

(56) References cited:
- WO-A1-2010/111073
- WO-A2-02/078775
- WO-A2-2006/127356
- GB-A- 2 396 426
- US-A- 5 365 922
- US-A1- 2010 224 192
- US-B1- 7 225 809

## Description

### BACKGROUND

### 1. Field

The present disclosure pertains to a method and apparatus controlling a ventilation therapy device, and, in particular, using physiological parameters, such as conveyed via a (patient) monitor signals and/or other physiological monitor signals, for closed-loop control of respiratory therapy regimens provided through a ventilator.

### 2. Description of the Related Art

It is well known to control a ventilator based on real-time measurements of gas parameters, such as pressure, flow, volume, gas mix, and/or other gas parameters, of the pressurized flow of breathable gas delivered to a subject through the ventilator. It is well known that patient monitors provide a variety of useful real-time information related to physiological parameters, such as heart rate, blood pressure, cardiac output, oxygen saturation, end-tidal carbon dioxide, and/or other physiological parameters of a subject.

US 7 225 809 B1, GB 2 396 426 A, US 5,365,922 A, WO 2006/127356 A2, WO 02/078775 A2 and WO 2010/224192 A1 disclose apparatus controlling a ventilation therapy device.

US7225809 discloses a pressure support system with a controller, wherein signals from various sensors may be provided to the controller. GB2396426 discloses an artificial respiration system communicating with an electrical impedance imaging system.

### SUMMARY

Accordingly, it is an object of the present invention to provide a system comprising a ventilator and a patient monitor device as defined in the appended claim 1. According to a preferred embodiment, the ventilator comprises a pressure generator, a subject interface, a communications transceiver, and one or more processors. The pressure generator may be configured to generate a pressurized flow of breathable gas for delivery to the airway of a subject. The subject interface may be configured to interface between the pressure generator and a breathing circuit to deliver the pressurized flow of breathable gas to an artificial airway (*e.g*. endotracheal or tracheostomy tubes) or the natural airway of the subject through the outlet. The communications transceiver may be configured to receive communication signals from a patient monitor device, the communication signals comprising information related to physiological parameters of the subject, the patient monitor device being a separate and discrete device from the ventilator. A plurality of ventilation sensors are configured to generate one or more output signals conveying measurements related to respiratory parameters of the subject.

A communications transceiver is configured to receive communication signals from the patient monitor device. The one or more processors may be configured to execute processing modules, including a control module and a regimen adjustment module. The control module may be configured to control the pressure generator such that one or more gas parameters of the pressurized flow of breathable gas are varied over time in accordance with a respiratory therapy regimen. The regimen adjustment module may be configured to determine adjustments to respiratory therapy regimens based on the communication signals received from the patient monitor device such that the physiological parameters indicated in the communications signals are used for closed-loop control to adjust the respiratory therapy regimen. The patient monitor device includes one or more patient monitor sensors. The patient monitor device is a separate and discrete device from the ventilator. The patient monitor sensors are configured to generate output signals conveying information related to the physiological parameters of the subject. The processing modules further comprising a parameter determination module configured to determine one or more parameters from the output signals generated by the plurality of ventilation sensors and one or more patient monitor sensors. Executing of the processing modules is based on the simultaneous use of output signals generated by the ventilation sensors and the patient monitor sensors.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 schematically illustrates a ventilator device according to one or more embodiments; and
FIG. 2 illustrates a method for operating a ventilator device.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, *i.e*., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e.*, a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

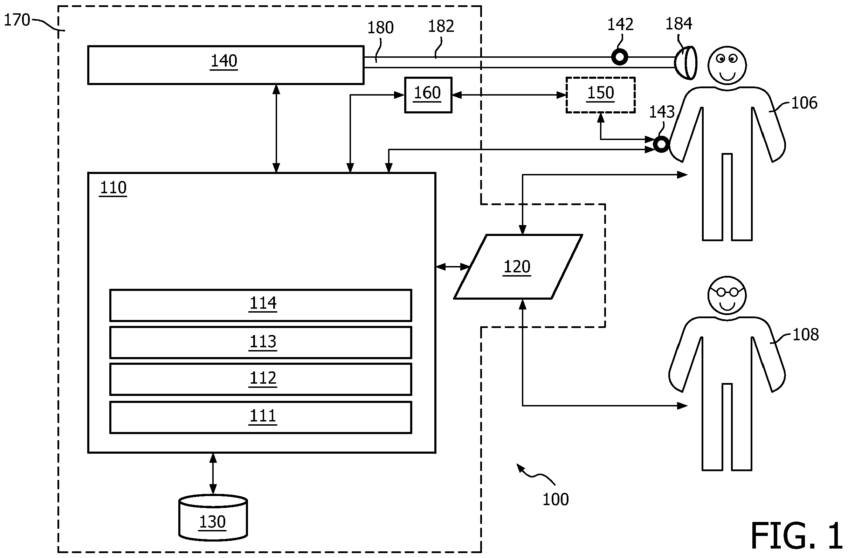

FIG. 1 illustrates a ventilator device 100 configured to deliver respiratory therapy to a subject 106. In particular, ventilator device 100 may be configured to mechanically ventilate subject 106 by providing a pressurized flow of breathable gas to an artifical airway of subject 106 or to the natural airway of subject 106 to inflate and/or deflate the lungs of subject 106. In some embodiments, the respiratory therapy regimen is configured to support the breathing of subject 106. In some embodiments, the respiratory therapy regimen is configured to cause subject 106 to breathe without any respiratory effort from subject 106. Ventilator device 100 may be configured such that the respiratory therapy regimen is controlled in a closed-loop manner using one or more physiological parameters, thus providing real-time control of oxygenation, ventilation, pressure support, and/or other services related to ventilation.

Ventilator device 100 may include one or more of a pressurized flow generator 140, a subject interface 180 (*i.e.* outlet for gas output and/or a combination of outlet and inlet), a subject interface appliance 184, a communications transceiver 160, one or more processors 110, and/or other components. Ventilator device 100 may operate in conjunction with a patient monitor device 150, which is a separate and discrete device from ventilator device 100, as indicated by the dotted box representing patient monitor device 150 in FIG. 1.

Patient monitor device 150 may be configured to monitor one or more physiological parameters of subject 106. In some embodiments, patient monitor device 150 may comprise a stand-alone physiological monitor rather than a standard patient monitor. Patient monitor device 150 may include, for example, a conventional monitoring device that measures a subject's vital signs and displays the data obtained through the measurements on an electronic display and/or transmits the data on a monitoring network. The physiological parameters may include one or more of blood pressure, heart rate, pulse oximetry, blood gas level, ECG, EEG, body temperature, (end-tidal) carbon dioxide concentration, parameters indicating pending cardiac conditions, cardiac output, sedation index and/or other physiological parameters. By way of non-limiting example, patient monitor device 150 may include one or more of the IntelliVue patient monitors by Philips (*e.g*., IntelliVue MP90, MMX800, MMS X2), SureSigns patient monitors by Philips (*e.g*., SureSigns VM8), and/or other patient monitoring devices. Patient monitor device 150 may include one or more of an electronic display, one or more patient monitor sensors 143, a user interface, one or more processors, and/or other components.

Patient monitor sensors 143 are configured to generate output signals conveying information related to physiological parameters, as described above, of subject 106. Patient monitor sensors 143 may be integrated and/or coupled to patient monitor device 150. Alternatively, and/or simultaneously, patient monitor sensors 143 may be separate and discrete from patient monitor device 150, and configured such that the output signals generated by patient monitor sensors 143 may be transmitted to patient monitor device 150 and/or ventilator device 100 (or a component thereof). This transmission may be accomplished via wired and/or wireless communication media. The illustration of patient monitor sensor 143 as including a single member in FIG. 1 is not intended to be limiting.

Pressure generator 140 of ventilator device 100 may be integrated, combined, coupled, or connected with an airway pressure device. Respiratory therapy may recommend delivery of a pressurized flow of breathable gas to the airway of a subject, providing one or more inhalation pressure, flow, and/or volume levels during the inhalation phase, and one or more exhalation pressure, flow, and/or volume levels during the exhalation phase. Any pressure level during an inhalation phase may be referred to as an inhalation pressure level, though such a pressure level need not be constant throughout the inhalation phase. In addition to alternating between multiple levels, the inhalation pressure level may ramp up or down according to a predetermined slope (absolute and/or relative, e.g. dependent on breathing rate) for any specified section of a phase. The flow level during inhalation can follow a predetermined flow waveform like a square or ramp. Similar features may be available for exhalation phases. The pressure and/or flow levels may be either predetermined and fixed, follow a predetermined dynamic characteristic, or they may dynamically change breath-to-breath or over several breaths.

Pressure generator 140 of ventilator device 100 may be configured to provide a pressurized flow of breathable gas to the airway of subject 106. Subject 106 may or may not initiate one or more phases of respiration. Ventilatory support may be implemented as a higher and lower positive pressure of a (multi-level) PAP device. For example, to support inspiration, the pressure of the pressurized flow of breathable gas may be adjusted to an inspiratory pressure and/or may be adjusted to a flow level. Alternatively, and/or simultaneously, to support expiration, the pressure of the pressurized flow of breathable gas may be adjusted to an expiratory pressure. Other schemes for providing respiratory support (including Volume Control Ventilation (VCV), Pressure Control Ventilation(PCV), Airway Pressure Release Ventilation (APRV), Pressure Regulated Volume Control (PRVC), CPAP, BiPAP®, and/or other schemes) through the delivery of the pressurized flow of breathable gas are contemplated.

Ventilator device 100 may be configured such that one or more gas parameters of the pressurized flow of breathable gas are controlled in accordance with a therapeutic respiratory regimen for subject 106. The one or more gas parameters may include one or more of flow, volume, pressure, humidity, gas mix, velocity, acceleration, (intentional) gas leak, and/or other parameters. Ventilator device 100 may be configured to provide types of therapy including types of therapy where a subject performs inspiration and/or expiration of his/her own accord and/or where the device provides mandatory controlled breaths.

A pressurized flow of breathable gas may be delivered by pressure generator 140 to or near the airway of subject 106 via a subject interface 180 (also referred to herein as an outlet). Subject interface 180 may include a conduit 182, a subject interface appliance 184, and/or other components. Conduit 182 may be a single-limb or a dual-limb flexible length of hose, or other conduit, that places subject interface appliance 184 in fluid communication with pressure generator 140. Conduit 182 forms a flow path through which the pressurized flow of breathable gas is communicated between subject interface appliance 184 and pressure generator 140.

Subject interface appliance 184 of ventilator device 100 in FIG. 1 is configured to deliver the pressurized flow of breathable gas to the airway of subject 106. As such, subject interface appliance 184 may include any appliance suitable for this function. In certain embodiments, pressure generator 140 is a dedicated ventilation device and subject interface appliance 184 is configured to be removably coupled with another interface appliance being used to deliver respiratory therapy to subject 106. For example, subject interface appliance 184 is configured to engage with and/or be inserted into an endotracheal tube, a tracheotomy portal, and/or other interface appliances. In certain embodiments, subject interface appliance 184 is configured to engage the airway of subject 106 without an intervening appliance. In this embodiment, subject interface appliance 184 includes one or more of an endotracheal tube, a nasal cannula, a tracheotomy tube, a nasal mask, a nasal/oral mask, a full face mask, a total face mask, a partial rebreathing mask, or other interface appliances that communicate a flow of gas with an airway of a subject. The present disclosure is not limited to these examples, and contemplates delivery of the pressurized flow of breathable gas to subject 106 using any subject interface.

Referring to FIG. 1, ventilator device 100 may include electronic storage 130 comprising electronic storage media that electronically stores information. The electronic storage media of electronic storage 130 includes one or both of system storage/ventilator device storage that is provided integrally (*i.e*., substantially non-removable) with ventilator device 100 and/or removable storage that is removably connectable to ventilator device 100 via, for example, a port (*e.g.,* a USB port, a FireWire port, *etc*.) or a drive (*e.g*., a disk drive, *etc*.). Electronic storage 130 may include one or more of optically readable storage media (*e.g*., optical disks, *etc*.), magnetically readable storage media (*e.g.,* magnetic tape, magnetic hard drive, floppy drive, *etc*.), electrical charge-based storage media (*e.g*., EEPROM, RAM, *etc*.), solid-state storage media (*e.g*., flash drive, *etc*.), and/or other electronically readable storage media. Electronic storage 130 stores software algorithms, information determined by processor 110, information received via user interface 120, and/or other information that enables ventilator device 100 to function properly. For example, electronic storage 130 may record or store timing information (including duration of inhalation phases and exhalation phases as well as transitional moments therebetween), one or more (breathing) parameters and/or other parameters (as discussed elsewhere herein), pressure levels, information indicating whether the subject adequately complied with a prescribed respiratory therapy regimen, information indicating adequacy of treatment, and/or other information. Electronic storage 130 may be a separate component within ventilator device 100, or electronic storage 130 may be provided integrally with one or more other components of ventilator device 100 (*e.g*., processor 110).

Patient monitor device 150 may include electronic storage having substantially similar functionality and capability as electronic storage 130, though pertaining to patient monitor 150 rather than ventilator device 100.

User interface 120 of ventilator device 100 in FIG. 1 is configured to provide an interface between ventilator device 100 and a user (*e.g*., user 108, subject 106, a caregiver, a therapy decision-maker, *etc*.) through which the user can provide information to and receive information from ventilator device 100. This enables data, results, and/or instructions and any other communicable items, collectively referred to as "information," to be communicated between the user and ventilator device 100. Examples of interface devices suitable for inclusion in user interface 120 include a keypad, buttons, switches, a keyboard, knobs, levers, a display screen, a touch screen, smartphones, smart touchpads, speakers, a microphone, an indicator light, an audible alarm, and a printer. Information is *e.g*. provided to subject 106 by user interface 120 in the form of auditory signals, visual signals, tactile signals, and/or other sensory signals.

It is to be understood that other communication techniques, either hard-wired or wireless, are also contemplated herein as user interface 120. For example, in one embodiment, user interface 120 is integrated with a removable storage interface provided by electronic storage 130. In this example, information is loaded into ventilator device 100 from removable storage (*e.g*., a smart card, a flash drive, a removable disk, *etc*.) that enables the user(s) to customize the implementation of ventilator device 100. Other exemplary input devices and techniques adapted for use with ventilator device 100 as user interface 120 include, but are not limited to, an RS-232 port, RF link, an IR link, modem (telephone, cable, Ethernet, internet or other), mobile phone and other wireless communication channels. In short, any technique for communicating information with ventilator device 100 is contemplated as user interface 120.

Patient monitor device 150 may include a user interface having substantially similar functionality and capability as user interface 120, though pertaining to patient monitor 150 rather than ventilator device 100.

Ventilation sensors 142 of ventilator device 100 in FIG. 1 is configured to generate one or more output signals conveying measurements related to the ventilation, oxygen saturation, and/or end-tidal carbon dioxide of subject 106. The measurements may relate to gas parameters and/or other parameters. Gas parameters may include flow, pressure, humidity, velocity, acceleration, and/or other gas parameters related to the pressurized flow of breathable gas delivered to subject 106. Output signals may convey measurements related to respiratory parameters. Ventilation sensor(s) 142 may be in fluid communication with conduit 182 and/or subject interface appliance 184.

The illustration of ventilation sensor 142 as including a single member in FIG. 1 is not intended to be limiting. In certain embodiments ventilation sensor 142 includes a plurality of sensors operating as described above by generating output signals conveying information related to parameters associated with the gas breathed by subject 106, the delivery of the gas to subject 106, oxygen saturation of subject 106, and/or end-tidal carbon dioxide of subject 106. Alternatively, and/or simultaneously, a parameter may be related to a mechanical unit of measurement of a component of ventilator device 100 such as rotor speed, motor speed, blower speed, fan speed, or a related measurement that serves as a proxy for any of the breathing parameters listed herein through a previously known/calibrated mathematical relationship. Resulting signals or information from sensor 142 may be transmitted to processor 110, user interface 120, electronic storage 130, and/or other components of ventilator device 100. This transmission can be wired and/or wireless.

Communications transceiver 160 of ventilator device 100 in FIG. 1 is configured to receive communication signals from patient monitor 150, which is a separate and discrete device from ventilator device 100. The communication signals may comprise information related to physiological parameters of subject 106. The communication signals may comprise the same signals and/or information as one or more patient monitor sensors 143. Alternatively and/or simultaneously, the communication signals may comprise data that is determined and/or generated by patient monitor device 150 based on output signals from patient monitor sensors 143. Such data may for example include minimum, maximum, mean, and/or average measured values, trend data for a particular patient monitor sensor 143 and/or a physiological parameter derived thereof, *e.g*. within a configurable period such as a minute, an hour, a day, a week, and/or another predetermined period. Communication signals may comprise information based on multiple signals and/or multiple physiological parameters derived therefrom.

In some embodiments, communications transceiver 160 may be configured to transmit signals, information, and/or alarms to patient monitor 150, *e.g.* for presentation on the user interface and/or the electronic display of patient monitor device 150. For example, a component of ventilator device 100 may determine a (real-time and/or continuous) parameter that is displayed on patient monitor 150, after transmission through communications transceiver 160. Such a parameter may be displayed along with other parameters based on output signals from patient monitor sensors 143.

Processor 110 of ventilator device 100 in FIG. 1 is configured to provide information processing capabilities within ventilator device 100. As such, processor 110 includes one or more of a digital processor, an analog processor, a digital circuit designed to process information, an analog circuit designed to process information, a state machine, and/or other mechanisms for electronically processing information. Although processor 110 is shown in FIG. 1 as a single entity, this is for illustrative purposes only. In some embodiments, processor 110 includes a plurality of processing units.

Patient monitor device 150 may include one or more processors having substantially similar functionality and capability as processor 110, though pertaining to patient monitor 150 (and its components) rather than ventilator device 100 (and its components).

As is shown in FIG. 1, processor 110 is configured to execute one or more computer program modules. The one or more computer program modules include one or more of a parameter determination module 111, a control module 112, a regimen adjustment module 113, an alarm module 114, and/or other modules. Processor 110 may be configured to execute modules 111, 112, 113, and/or 114 by software; hardware; firmware; some combination of software, hardware, and/or firmware; and/or other mechanisms for configuring processing capabilities on processor 110. Processor 110 may be configured to execute one or more modules that receive and/or process the output signals generated by patient monitor sensors 143 and/or ventilation sensors 142. Based on the output signals generated by patient monitor sensors 143, the processing modules may monitor the physiological parameters of subject 106. The processing modules are further configured to present information related to at least one physiological parameter. Processor 110 may be configured to execute one or more modules that receive and/or process signals and/or information obtained from patient monitor device 150.

It should be appreciated that although modules 111, 112, 113, and 114 are illustrated in FIG. 1 as being co-located within a single processing unit, in embodiments in which processor 110 includes multiple processing units, one or more of modules 111, 112, 113, and/or 114 may be located remotely from the other modules. The description of the functionality provided by the different modules 111, 112, 113, and/or 114 described below is for illustrative purposes, and is not intended to be limiting, as any of modules 111, 112, 113, and/or 114 may provide more or less functionality than is described. For example, one or more of modules 111, 112, 113, and/or 114 may be eliminated, and some or all of its functionality may be provided by other ones of modules 111, 112, 113, and/or 114. Note that processor 110 may be configured to execute one or more additional modules that may perform some or all of the functionality attributed below to one of modules 111, 112, 113, and/or 114. Note that one or more of modules 111, 112, 113, and 114, and/or some or all of their respective functionalities may be provided by patient monitor device 150 (or components thereof).

Parameter determination module 111 is configured to determine one or more gas parameter, breathing parameters, and/or other parameters from the output signals generated by patient monitor sensors 143 and/or ventilation sensors 142. Gas parameters of the (pressurized) flow of breathable gas may include one or more of (peak) flow, pressure, temperature, humidity, velocity, acceleration, gas composition (*e.g*. concentration(s) of one or more constituents), thermal energy dissipated, (intentional) gas leak, and/or other gas parameters related to the (pressurized) flow of breathable gas. Breathing parameters may be derived from the one or more gas parameters, and include one or more of tidal volume of the breathing of the subject, retrograde volume, respiratory rate, breathing period, inhalation time or period, exhalation time or period, inhalation volume, exhalation volume, peak flow, flow rate of the breathing of the subject, respiration flow curve shape, transition time from inhalation to exhalation and/or vice versa, transition time from peak inhalation flow rate to peak exhalation flow rate and/or vice versa, respiration pressure curve shape, pressure-volume loops, flow-volume loops, (intentional) gas leak, and/or other breathing parameters. Some or all of the stated functionality of parameter determination module 111 may be incorporated or integrated into other computer program modules of processor 110 and/or patient monitor device 150.

Control module 112 is configured to control pressure generator 140 such that one or more gas parameters of the pressurized flow of breathable gas are varied over time in accordance with a respiratory therapy regimen. Control module 112 may be configured to control pressure generator 140 to provide the pressurized flow of breathable gas at inhalation pressure levels during inhalation phases, and at exhalation pressure levels during exhalation phases. Parameters determined by parameter determination module 111 (and/or received through patient monitor sensors 143) may be used by control module 112, *e.g.* in a feedback manner, to adjust ventilator settings/operations. Alternatively, and/or simultaneously, signals and/or information received by communications transceiver 160 may be used by control module 112, *e.g.* in a feedback manner, to adjust ventilator settings/operations. Control module 112 may be configured to time its operations relative to the transitional moments in the breathing cycle of a subject and over multiple breath cycles.

Regimen adjustment module 113 is configured to determine adjustments to the respiratory therapy regimen. Adjustments may be based on communication signals received from patient monitor device 150 such that the physiological parameters indicated in the communication signals are used as closed-loop control parameters in adjusting the respiratory therapy regimen. Alternatively, and/or simultaneously, adjustments may be based on parameters determined by parameter determination module 111. Once determined, the adjustments may be implemented by regimen adjustment module 113, control module 112, and/or other components of ventilator device 100. In some embodiments, the communication signals are related in a known manner to the parameters determined by parameter determination module 111. In such a case, the determination by regimen adjustment module 113 is more robust than a similar determination for a ventilator that lacks the information transmitted from a patient monitor device. In some embodiments, the communication signals are unrelated (or not related in a known manner) to the parameters determined by parameter determination module 111. In such a case, the determination by regimen adjustment module 113 is based on more data (and different types of data) than a similar determination for a ventilator that lacks the information transmitted from a patient monitor device.

Alarm module 114 is configured to receive information and/or parameters from any component of ventilator device 100, any component of patient monitor 150, and/or any patient monitor sensor 143. Alarm module 114 may be configured to monitor any received information and/or parameter for alarm conditions, including exceeding an upper or lower limit. Alarm conditions may be determined for mean or average values. Alarm conditions may be determined on a logical combination of multiple parameters. For example, an alarm condition could be programmed, *e.g*. through user interface 120, as a logical conjunction of a) a heart rate exceeding some predetermined value *X*, and, simultaneously, b) the respiratory rate exceeding some predetermined value *Y*. Additional logical conditions are considered, as are consecutive and/or nested alarm conditions that follow each other in time, such as a certain duration with an average heart rate exceeding *X*, followed within a certain duration by an occurrence of a respiratory rate exceeding Y.

Alarm module 114 may be configured to transmit, *e.g*. through communications transceiver 160, an occurrence of an alarm and/or a determination of an alarm condition to patient monitor device 150 for presentation to a user and/or subject.

Ventilator device 100 may include an external housing 170 that houses at least pressure generator 140 and one or more of processors 110. Patient monitor device 150 is outside of and physically separate from external housing 170.

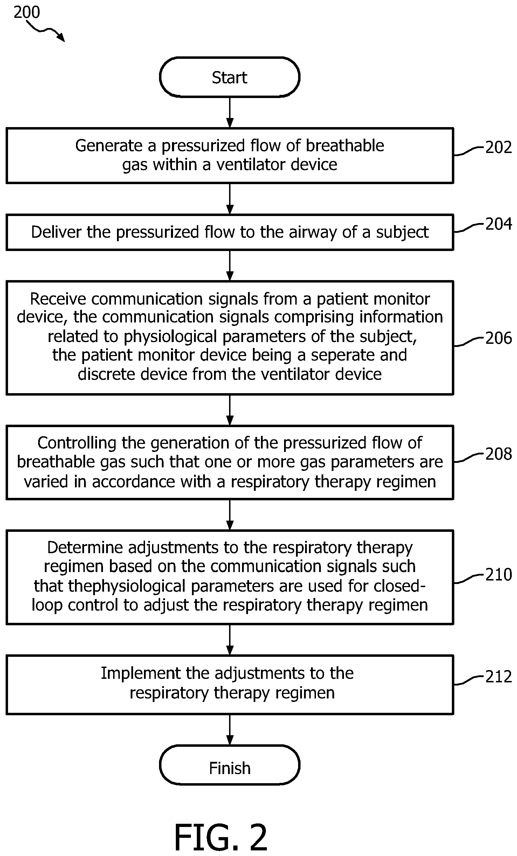

FIG. 2 illustrates a method 200 for operating a ventilator device. The operations of method 200 presented below are intended to be illustrative. In certain embodiments, method 200 may be accomplished with one or more additional operations not described, and/or without one or more of the operations discussed. Additionally, the order in which the operations of method 200 are illustrated in FIG. 2 and described below is not intended to be limiting.

In certain aspects, method 200 may be implemented in one or more processing devices (*e.g*., a digital processor, an analog processor, a digital circuit designed to process information, an analog circuit designed to process information, a state machine, and/or other mechanisms for electronically processing information). The one or more processing devices may include one or more devices executing some or all of the operations of method 200 in response to instructions stored electronically on an electronic storage medium. The one or more processing devices may include one or more devices configured through hardware, firmware, and/or software to be specifically designed for execution of one or more of the operations of method 200.

At an operation 202, a pressurized flow of breathable gas is generated within a ventilator. In one embodiment, operation 202 is performed by a pressure generator similar to or substantially the same as pressure generator 140 (shown in FIG. 1 and described above).

At an operation 204, the pressurized flow of breathable gas is delivered to the airway of a subject. In one embodiment, operation 204 is performed by an outlet or subject interface similar to or substantially the same as subject interface 180 (shown in FIG. 1 and described above).

At an operation 206, communication signals are received from a patient monitor device. The communication signals comprise information related to physiological parameters of the subject. The patient monitor device is a separate and discrete device from the ventilator device. In one embodiment, operation 206 is performed by a communications transceiver similar to or substantially the same as communications transceiver 160 (shown in FIG. 1 and described above).

At an operation 208, the generation of the pressurized flow of breathable gas is controlled such that one or more gas parameters of the pressurized flow of breathable gas are varied over time in accordance with a respiratory therapy regimen. In one embodiment, operation 208 is performed by a control module similar to or substantially the same as control module 112 (shown in FIG. 1 and described above).

At an operation 210, adjustments are determined to the respiratory therapy regimen based on the communication signals received from the patient monitor device such that the physiological parameters are used for closed-loop control to adjust the respiratory therapy regimen. In one embodiment, operation 210 is performed by a regimen adjustment module similar to or substantially the same as regimen adjustment module 113 (shown in FIG. 1 and described above).

At an operation 212, the adjustments to the respiratory therapy regimen are implemented. In one embodiment, operation 212 is performed by a regimen adjustment module 113 similar to or substantially the same as regimen adjustment module 113 (shown in FIG. 1 and described above).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although various embodiments have been described in detail for the purpose of illustration based on what is currently considered to be the most practical and preferred embodiments, it is to be understood that such detail is solely for that purpose and that the scope of this disclosure is not limited to the disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A system comprising a ventilator (100) and a patient monitor device (150), wherein the patient monitor device (150) is a separate and discrete device from the ventilator (100), the ventilator comprising:
a pressure generator (140) configured to generate a pressurized flow of breathable gas;
a subject interface (180) configured to interface between the pressure generator and a breathing circuit to deliver the pressurized flow of breathable gas to the airway of a subject (106) through the subject interface;
a communications transceiver (160) configured to receive communication signals from the patient monitor device, the communication signals comprising information related to physiological parameters of the subject;
a plurality of ventilation sensors (142) configured to generate one or more output signals conveying measurements related to respiratory parameters of the subject (106);
one or more processors (110) configured to execute processing modules, the processing modules comprising:
a control module (112) configured to control the pressure generator such that one or more gas parameters of the pressurized flow of breathable gas are varied over time in accordance with a respiratory therapy regimen; and
a regimen adjustment module (113) configured to determine adjustments to the respiratory therapy regimen based on the communication signals received from the patient monitor device such that the physiological parameters indicated in the communication signals are used for closed-loop control to adjust the respiratory therapy regimen,
**characterized in that**
the patient monitor device (150) includes one or more patient monitor sensors (143); wherein the patient monitor sensors (143) are configured to generate output signals conveying information related to the physiological parameters of the subject (106);
the processing modules further comprising a parameter determination module configured to determine one or more parameters from the output signals generated by the plurality of ventilation sensors (142) and one or more patient monitor sensors (143), wherein executing of the processing modules is based on the simultaneous use of output signals generated by the ventilation sensors (142) and the patient monitor sensors (143).

2. The system of claim 1, wherein the adjustments to the respiratory therapy regimen comprise adjustments to one or more of the gas parameters of the pressurized flow of breathable gas that are varied over time in accordance with the respiratory therapy regimen.

3. The system of claim 1, wherein the respiratory therapy regimen is determined such that delivery of the pressurized flow of breathable gas to the airway of the subject controls one or more breathing parameters of the subject, and wherein the regimen adjustment module is configured to determine adjustments to the respiratory therapy regimen such that a breathing parameter of the subject is controlled based on the physiological parameters indicated in the communication signals.

4. The system of claim 1, wherein the regimen adjustment module is configured to determine adjustments to the respiratory therapy regimen further based on the physiological parameters of the subject, wherein the adjustments pertain to control of one or more of nebulization, humidification, or inexsufflation.

5. The system of claim 1, further comprising an external housing (170) that houses at least the pressure generator and the one or more processors.

## Patentansprüche

1. System, umfassend ein Beatmungsgerät (100) und eine Patientenüberwachungsvorrichtung (150), wobei die Patientenüberwachungsvorrichtung (150) eine getrennte und eigenständige Vorrichtung gegenüber dem Beatmungsgerät (100) ist, wobei das Beatmungsgerät Folgendes umfasst:
einen Druckgenerator (140), der konfiguriert ist, um eine unter Druck stehende Strömung von Atemgas zu generieren;
eine Personenschnittstelle (180), die konfiguriert ist, um zwischen dem Druckgenerator und einem Beatmungskreislauf eine Schnittstelle zu bilden, um die unter Druck stehende Strömung von Atemgas an die Atemwege einer Person (106) durch die Personenschnittstelle hindurch abzugeben;
einen Kommunikations-Transceiver (160), der konfiguriert ist, um Kommunikationssignale von der Patientenüberwachungsvorrichtung zu empfangen, wobei die Kommunikationssignale Informationen über physiologische Parameter der Person umfassen;
eine Vielzahl von Beatmungssensoren (142), die konfiguriert sind, um ein oder mehrere Ausgangssignale zu generieren, die Messungen bezüglich der Atemparameter der Person (106) übermitteln;
einen oder mehrere Prozessoren (110), der bzw. die konfiguriert ist bzw. sind, um Verarbeitungsmodule durchzuführen, wobei die Verarbeitungsmodule Folgendes umfassen:
ein Steuermodul (112), das konfiguriert ist, um den Druckgenerator derart zu steuern, dass ein oder mehrere Gasparameter der unter Druck stehenden Strömung von Atemgas im Verlauf der Zeit in Übereinstimmung mit einer Atemtherapiebehandlung variiert wird bzw. werden; und
ein Behandlungsanpassungsmodul (113), das konfiguriert ist, um Anpassungen an der Atemtherapiebehandlung basierend auf den Kommunikationssignalen, die von der Patientenüberwachungsvorrichtung empfangen werden, zu bestimmen, so dass die physiologischen Parameter, die in den Kommunikationssignalen angegeben sind, für die zur Regelung verwendet werden, um die Atemtherapiebehandlung anzupassen,
**dadurch gekennzeichnet, dass**
die Patientenüberwachungsvorrichtung (150) einen oder mehrere Patientenmonitorsensoren (143) umfasst; wobei die Patientenmonitorsensoren (143) konfiguriert sind, um Ausgangssignale zu generieren, die Informationen bezüglich der physiologischen Parameter der Person (106) übermitteln;
die Verarbeitungsmodule ferner ein Parameterbestimmungsmodul umfassen, das konfiguriert ist, um einen oder mehrere Parameter aus den Ausgangssignalen, die durch die Vielzahl von Beatmungssensoren (142) und einen oder mehrere Patientenüberwachungssensoren (143) generiert werden, zu bestimmen, wobei das Ausführen der Verarbeitungsmodule auf der gleichzeitigen Verwendung von Ausgangssignalen, die durch die Beatmungssensoren (142) und die Patientenüberwachungssensoren (143) generiert werden, basiert.

2. System nach Anspruch 1, wobei die Anpassungen an der Atemtherapiebehandlung Anpassungen an einem oder mehreren der Gasparameter der unter Druck stehenden Strömung von Atemgas, der bzw. die im Verlauf der Zeit in Übereinstimmung mit der Atemtherapiebehandlung variiert wird bzw. werden, umfassen.

3. System nach Anspruch 1, wobei die Atemtherapiebehandlung derart bestimmt wird, dass die Abgabe der unter Druck stehenden Strömung von Atemgas an die Atemwege der Person einen oder mehrere Atemparameter der Person steuert, und wobei das Behandlungsanpassungsmodul konfiguriert ist, um Anpassungen an der Atemtherapiebehandlung derart zu bestimmen, dass ein Atemparameter der Person basierend auf den physiologischen Parametern, die in den Kommunikationssignalen angegeben sind, gesteuert wird.

4. System nach Anspruch 1, wobei das Behandlungsanpassungsmodul konfiguriert ist, um Anpassungen an der Atemtherapiebehandlung ferner basierend auf den physiologischen Parametern der Person zu bestimmen, wobei die Anpassungen das Steuern einer oder mehrerer von Zerstäubung, Befeuchtung oder Ein-/Ausblasung betreffen.

5. System nach Anspruch 1, ferner umfassend ein externes Gehäuse (170), das mindestens den Druckgenerator und den einen oder die mehreren Prozessoren aufnimmt.

## Revendications

1. Système comprenant un ventilateur (100) et un dispositif de surveillance de patient (150), dans lequel le dispositif de surveillance de patient (150) est un dispositif distinct et isolé du ventilateur (100), le ventilateur comprenant :
un générateur de pression (140) configuré pour générer un écoulement sous pression de gaz respirable ;
une interface sujet (180) configurée pour faire office d'interface entre le générateur de pression et un circuit respiratoire pour distribuer l'écoulement sous pression de gaz respirable aux voies aériennes d'un sujet (106) à travers l'interface sujet ;
un émetteur-récepteur de communication (160) configuré pour recevoir des signaux de communication provenant du dispositif de surveillance de patient, les signaux de communication comprenant des informations concernant des paramètres physiologiques du sujet;
une pluralité de capteurs de ventilation (142) configurés pour générer un ou plusieurs signaux de sortie acheminant des mesures concernant des paramètres respiratoires du sujet (106) ;
un ou plusieurs processeurs (110) configurés pour exécuter des modules de traitement, les modules de traitement comprenant :
un module de commande (112) configuré pour commander le générateur de pression de sorte qu'un ou plusieurs paramètres de gaz de l'écoulement sous pression de gaz respirable varient au fil du temps en fonction d'un régime de thérapie respiratoire ; et
un module d'ajustement de régime (113) configuré pour déterminer des ajustements du régime de thérapie respiratoire sur la base des signaux de communication reçus en provenance du dispositif de surveillance de patient de sorte que les paramètres physiologiques indiqués dans les signaux de communication soient utilisés en commande en boucle fermée pour ajuster le régime de thérapie respiratoire,
**caractérisé en ce que**
le dispositif de surveillance de patient (150) inclut un ou plusieurs capteurs de surveillance de patient (143) ; dans lequel les capteurs de surveillance de patient (143) sont configurés pour générer des signaux de sortie acheminant des informations concernant les paramètres physiologiques du sujet (106) ;
les modules de traitement comprenant en outre un module de détermination de paramètres configuré pour déterminer un ou plusieurs paramètres provenant des signaux de sortie générés par la pluralité de capteurs de ventilation (142) et d'un ou plusieurs capteurs de surveillance de patient (143), dans lequel l'exécution des modules de traitement est basée sur l'utilisation simultanée des signaux de sortie générés par les capteurs de ventilation (142) et les capteurs de surveillance de patient (143).

2. Système selon la revendication 1, dans lequel les ajustements du régime de thérapie respiratoire comprennent l'ajustement d'un ou plusieurs des paramètres de gaz de l'écoulement sous pression de gaz respirable qui varient au fil du temps en fonction du régime de thérapie respiratoire.

3. Système selon la revendication 1, dans lequel le régime de thérapie respiratoire est déterminé de sorte qu'une distribution de l'écoulement sous pression de gaz respirable aux voies aériennes du sujet commande un ou plusieurs paramètres respiratoires du sujet, et dans lequel le module d'ajustement de régime est configuré pour déterminer des ajustements du régime de thérapie respiratoire de sorte qu'un paramètre respiratoire du sujet soit commandé sur la base des paramètres physiologiques indiqués dans les signaux de communication.

4. Système selon la revendication 1, dans lequel le module d'ajustement de régime est configuré pour déterminer des ajustements du régime de thérapie respiratoire en outre sur la base des paramètres physiologiques du sujet, dans lequel les ajustements portent sur la commande d'une ou plusieurs parmi la nébulisation, l'humidification, ou l'insufflation-exsufflation.

5. Système selon la revendication 1, comprenant en outre un boîtier externe (170) qui loge au moins le générateur de pression et les un ou plusieurs processeurs.
